(19) Europäisches Patentamt
European Patent Office
Office européen des brevets

(11) **EP 4 640 840 A1**

(12) **EUROPEAN PATENT APPLICATION**
published in accordance with Art. 153(4) EPC

(43) Date of publication:
29.10.2025 Bulletin 2025/44

(21) Application number: 24842400.4

(22) Date of filing: 17.07.2024

(51) International Patent Classification (IPC):
*C12P 19/56* (2006.01)     *C12P 19/18* (2006.01)
*C12P 19/14* (2006.01)     *C12P 19/22* (2006.01)
*C07H 1/06* (2006.01)      *C07H 3/02* (2006.01)
*C07H 15/256* (2006.01)

(52) Cooperative Patent Classification (CPC):
C07H 1/06; C07H 3/02; C07H 15/256; C12P 19/14;
C12P 19/18; C12P 19/22; C12P 19/56

(86) International application number:
PCT/CN2024/106037

(87) International publication number:
WO 2025/016411 (23.01.2025 Gazette 2025/04)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 18.07.2023  CN 202310879842
25.09.2023  CN 202311239776
29.12.2023  CN 202311848635

(71) Applicant: Dongtai Haorui Biotechnology Co., Ltd
Yancheng, Jiangsu 224237 (CN)

(72) Inventors:
• ZHU, Liping
Yancheng, Jiangsu 224237 (CN)

• HE, Dongsheng
Yancheng, Jiangsu 224237 (CN)
• GUO, Xiaojie
Yancheng, Jiangsu 224237 (CN)
• LIU, Hao
Yancheng, Jiangsu 224237 (CN)
• CAO, Xinxin
Yancheng, Jiangsu 224237 (CN)
• JU, Min
Yancheng, Jiangsu 224237 (CN)
• ZANG, Pengfei
Yancheng, Jiangsu 224237 (CN)

(74) Representative: Winter, Brandl - Partnerschaft
mbB
Alois-Steinecker-Straße 22
85354 Freising (DE)

(54) **METHOD FOR PREPARING GLUCOSYL STEVIOSIDE USING MOTHER LIQUOR SUGAR, SEPARATING AND PURIFYING GLUCOSYL STEVIOSIDE AND RECOVERING GLUCOSE**

(57) The present disclosure discloses a method for preparing a glucosylated steviol glycoside (GSG) with mother liquor sugar (MLS) and a method for separating and purifying GSG and recovering glucose, and belongs to the technical field of food additives. The present disclosure provides a method for preparing GSG with MLS, where GSG is prepared with MLS as a raw material. The method for preparing GSG with MLS expands the utilization pathways for MLS, and fills the technical gap in producing GSGs with MLS as a raw material. Moreover, a product of this method has advantages such as excellent taste and high total glycoside content. The present disclosure also provides a method for purifying GSG, where dextrin is recycled. The method for purifying GSG reduces the emission of waste liquids, significantly decreases the energy consumption and cost of production, and improves the yield of high-purity GSGs. The present disclosure also provides a method for separating and purifying GSG and recovering glucose, which involves a simple process and easy operations. The method for separating and purifying GSG and recovering glucose not only leads to high-purity GSGs, but also achieves the recycling of the unreacted substrates, thereby reducing the cost.

EP 4 640 840 A1

**Description**

**CROSS REFERENCE TO RELATED APPLICATIONS**

The present application claims priority to the Chinese Patent Application No. 202311848635.2 filed to the China National Intellectual Property Administration (CNIPA) on December 29, 2023 and entitled "METHOD FOR PREPARING GLUCOSYLATED STEVIOL GLYCOSIDE (GSG) WITH MOTHER LIQUOR SUGAR (MLS)", the Chinese Patent Application No. 202310879842.8 filed to the China National Intellectual Property Administration (CNIPA) on July 18, 2023 and entitled "METHOD FOR PURIFYING GLUCOSYLATED STEVIOL GLYCOSIDE (GSG)", and the Chinese Patent Application No. 202311239776.4 filed to the China National Intellectual Property Administration (CNIPA) on September 25, 2023 and entitled "METHOD FOR SEPARATING AND PURIFYING GLUCOSYLATED STEVIOL GLYCOSIDE (GSG) AND RECOVERING GLUCOSE", which are incorporated herein by reference in their entireties.

**TECHNICAL FIELD**

The present disclosure relates to the technical field of food additives, and particularly relates to a method for preparing a glucosylated steviol glycoside (GSG) with mother liquor sugar (MLS) and a method for separating and purifying GSG and recovering glucose.

**BACKGROUND**

Steviol glycosides are natural sweeteners extracted from the *Stevia rebaudiana* plant of the *Asteraceae* family. Steviol glycosides are characterized by high sweetness and low caloric values. Steviol glycosides are 100 to 300 times sweeter than sucrose, but provide a caloric value only 1/300 of a caloric value of sucrose. Steviol glycosides exhibit both sweetness and bitterness, which both are related to the number of glycosyl groups attached to the main moiety and the types of glycosidic bonds among the glycosyl groups.

Mother liquid sugar (MLS) from the production of stevia glycosides is a powdered solid produced by spray-drying a mother liquor left after high-purity rebaudioside A (RA) and stevioside (STV) are crystallized and recovered through solution extraction. Generally, MLS from the production of stevia glycosides is sold as a low-cost sweetener. In addition, steviol glycosides (such as RA, STV, and rebaudioside C (RC)) are separated from MLS by a resin method (such as mixed resin-based chromatography and modified resin-based chromatography), and then further utilized. However, MLS has a relatively-low total glucoside content, and is an unqualified stevia product. MLS also includes residual polyphenol and flavone impurities, which exacerbate the bitterness of MLS and affect the recovery of glycosides.

GSGs are produced as follows: steviol glycosides are mixed with dextrin, the steviol glycosides are glycosylated through enzymatic catalysis such that one or more glucosyl groups are covalently bonded to each steviol glycoside molecule, and then evaporative concentration and spray-drying are conducted. GSGs are modified products with improved taste and reduced bitterness, and have been approved for use in countries such as the United States and China. During the actual production process, because the dextrose equivalent (DE) value of dextrin is related to the degree of hydrolysis and the content of substances such as dextrin and polysaccharides is positively correlated with the viscosity of a product, the glycosylation conversion efficiency is low.

As a result, how to efficiently prepare GSGs from MLS has become an urgent problem to be solved.

GSGs are flavoring agents widely used in the food and beverage industry. In industrial production, GSGs require purification to meet the high purity and cleanliness standards.

Currently, GSGs are commonly purified through ion-exchange chromatography. Ion-exchange chromatography is a separation technique based on the interaction of ions in a sample with a stationary phase. In ion-exchange chromatography columns, a stationary phase carries ionic functional groups capable of selectively adsorbing and releasing target components from a sample. For the purification of GSGs, strong cation-exchange resins are commonly adopted. After a concentrated solution is loaded on a strong cation-exchange resin column, under appropriate conditions, GSGs can be adsorbed on the resin while other irrelevant components flow away. Finally, under elution conditions, the GSGs adsorbed on the resin can be eluted to obtain a relatively pure product.

In the existing preparation and purification processes for GSGs, a large amount of the adjuvant dextrin is produced, and subsequently, the waste liquid needs to be treated before being discharged, which increases the energy consumption and cost of production.

The conversion process for GSGs is not a simple combination reaction (A + B = C). According to the properties of enzymes, there will be various combinations of sugar chains with glycosyl ligands, and GSGs with different numbers of glucosyl groups occur in a reaction system. Moreover, the conversion process is not easily controlled, and the reaction degree cannot reach 100%. As a result, the corresponding GSG product has a low purity, and needs to be purified.

The patent application No. 202010447914.8 provides a method for separating and purifying GSG. This method is specifically as follows: A material generated from the previous work section is treated with an ion exchange resin to remove cations in the material, then pumped at a rate of 0.5 m³/h to 1 m³/h into a simulated moving bed, and treated at 25°C to 40°C for 1 h to 2.5 h. The total time is calculated based on a feed rate. The corresponding material is collected at three intervals in a ratio of 1:3:1. The feed rate is controlled at 0.3 m³/h to 2 m³/h, the feed temperature is controlled at 20°C to 35°C, and the feed time is controlled at 0.5 h to 3 h. A material produced at 30 min to 90 min is pumped into a single-effect concentrator, and concentrated at a pressure of -0.6 MPa to -0.9 MPa and a temperature of 45°C to 60°C until a sugar concentration is 50% or more to produce a concentrate. The concentrate is then filtered, delivered to a concentrated sugar solution tank, and spray-dried. In the above patent, a material is decolorized with a resin and then separated in a simulated moving bed. Although high-purity GSGs can be produced by this method, there are high equipment requirements and costs.

## SUMMARY

Technical problems to be solved by the present disclosure: In view of the shortcomings in the prior art, the present disclosure provides a method for preparing GSG with MLS. The method for preparing GSG with MLS expands the utilization pathways for MLS, and fills the technical gap in producing GSGs with MLS. GSG prepared from MLS has a prominent taste, a high yield, and a high total glycoside content.

The present disclosure also provides a method for purifying GSG, which is obtained by optimizing and improving the purification process for GSGs, and achieves the effective recycling of the adjuvant dextrin. The method for purifying GSG not only reduces the emission of waste liquids and significantly lowers the energy consumption and cost of production, but also improves the yield of high-purity GSGs.

The present disclosure also provides a method for separating and purifying GSG and recovering glucose. The method for separating and purifying GSG and recovering glucose involves GSG dissolution, concentration, nanofiltration, flocculent settling, and cooling crystallization. The method for separating and purifying GSG and recovering glucose leads to high-purity GSGs, and achieves the recovery of glucose with a low cost.

To solve the above technical problems, the present disclosure adopts the following technical solutions:
A method for preparing GSG with MLS is provided, including the following steps:

(1) mixing the MLS, maltodextrin, and water, and stirring until the MLS and the maltodextrin are fully dissolved; and adding an enzyme, and allowing enzymatic hydrolysis to produce an enzymatic hydrolysate;

(2) adding activated carbon to the enzymatic hydrolysate obtained in the step (1) for impurity removal to produce an impurity-free solution; subjecting the impurity-free solution to enzyme inactivation and then filtration to produce a filtrate; and subjecting the filtrate to an adsorption treatment with a macroporous adsorption resin;

(3) conducting desorption with an acid-alcohol solution that is produced from an ethanol solution and a hydrochloric acid solution and has a pH of 1 to 3 to produce a desorption solution;

(4) adjusting a pH of the desorption solution to 5 to 6, and concentrating with a nanofiltration membrane to produce a concentrate; and

(5) adding a taste modifier to the concentrate, and spray-drying to produce the GSG.

As an improved technical solution, in the step (1), the MLS, the maltodextrin, and the water are in a mass ratio of 1:(1-3):(5-10).

As an improved technical solution, in the step (1), the enzyme is a mixture of a glucosyltransferase and an amylase; and the glucosyltransferase is α-cyclodextrin glucosyltransferase. Specifically, the glucosyltransferase is added in a form of a glucosyltransferase solution with a concentration of 0.8 wt% to 1.2 wt%; and the amylase is added in a form of an amylase solid with a concentration of 0.4 wt% to 0.6 wt%.

Further, 0.5 mL to 2 mL of the glucosyltransferase solution is added per 100 g of the MLS, and 0.1 g to 1 g of the amylase solid is added per 100 g of the MLS.

As an improved technical solution, in the step (1), the enzymatic hydrolysis is conducted for 36 h to 48 h at a temperature of 60°C to 80°C and a pH of 5.5 to 6.0.

As an improved technical solution, in the step (2), an amount of the activated carbon added is 1% to 3% of a mass of the enzymatic hydrolysate, and the impurity removal lasts for 1 h to 6 h.

As an improved technical solution, in the step (2), when the adsorption treatment is conducted with the macroporous adsorption resin, a feed volume of the filtrate is 30% to 60% of a volume of the macroporous adsorption resin, and a feed rate of the filtrate is 0.5 BV/h to 2 BV/h.

As an improved technical solution, before the desorption for a macroporous adsorption resin undergoing adsorption, the method further includes: washing the macroporous adsorption resin undergoing adsorption with purified water, where during the washing, an amount of the purified water is 1 BV to 3 BV of a volume of the macroporous adsorption resin undergoing adsorption, and a flow rate of the purified water is 1 BV/h to 3 BV/h.

As an improved technical solution, in the step (3), a concentration of the ethanol solution is 40 v/v% to 60 v/v% and a concentration of the hydrochloric acid solution is 0.3 wt% to 0.5 wt%; and during the desorption, a volume of the acid-alcohol solution is 2 BV to 4 BV of a volume of a macroporous adsorption resin undergoing adsorption, and a flow rate of the acid-alcohol solution is 1 BV/h to 3 BV/h.

As an improved technical solution, in the step (4), molecular weight cut-off of the nanofiltration membrane is 400 Da to 500 Da.

As an improved technical solution, in the step (5), the taste modifier is a composition of erythritol, sodium alginate, and valine in a mass ratio of 2:(2-4):(5-8); and an amount of the taste modifier added is 0.1% to 0.5% of a weight of a product in the concentrate.

Due to the above technical solutions, the present disclosure has the following beneficial effects:

(1) MLS from the production of stevia glycosides is characterized by a low total glycoside content. Due to the presence of residual polyphenol and flavone impurities, MLS exhibits obvious bitterness. The present disclosure innovatively prepares GSGs with MLS as a raw material and maltodextrin as a glucose-based substrate under the dual catalysis of a glucosyltransferase and an amylase, which reduces the bitterness of MLS. Then the impurity removal and deodorization are conducted with activated carbon, which further improves the taste of GSGs. Then, GSGs are enriched and separated through the adsorption of a macroporous adsorption resin. A resin undergoing adsorption is subjected to desorption with an acid-alcohol solution to produce a desorption solution, and a pH of the desorption solution is adjusted to 5 to 6, which ensures the stability of glycosides during the subsequent concentration process and improves the yield and quality. Further, GSGs are modified by adding a taste modifier to adjust the taste. Finally, a GSG product that has an excellent taste and a high total glycoside content and can be directly sold is produced through spray-drying.

(2) In the present disclosure, an amylase is added as an adjuvant on the basis of the existing glucosyltransferase, which addresses the issue that there is a low glucosyl transfer rate due to a low hydrolysis degree of dextrin, large quantities of dextrin and polysaccharides, and a high viscosity of a product. The combination of a glucosyltransferase with an amylase accelerates the hydrolysis of dextrin and the transfer of glucosyl, and improves the glucosyl transfer rate.

(3) In the present disclosure, a mixed solution of a hydrochloric acid solution and an ethanol solution is adopted for desorption, which achieves a prominent desorption effect, does not cause the partial loss of a product, and greatly improves the yield of a product. Moreover, in the present disclosure, before desorption, a resin undergoing adsorption is further washed with purified water, which not only allows the residual unabsorbed material in the resin to re-enter the resin for absorption, but also can effectively remove the monosaccharides and oligosaccharides produced during a glucosyl transfer process, thereby enhancing the desorption effect and product yield.

(4) In the present disclosure, through a reasonable process, MLS from the production of stevia glycosides is directly converted into a market-ready GSG product. Compared with the existing technologies, the present disclosure makes full use of MLS, expands the utilization pathways for MLS, and fills the technical gap in producing GSGs with MLS.

To solve the above technical problems, the present disclosure adopts the following technical solutions:
A method for purifying GSG is provided, including the following steps:

(1) dissolving a crude GSG product in an ethanol solution to produce a GSG-containing ethanol solution; subjecting the GSG-containing ethanol solution to an adsorption treatment with a macroporous adsorption resin, and collecting an effluent; and conducting desorption with an acid aqueous solution and a high-purity ethanol solution successively, and collecting an acid-water desorption solution and a high-purity ethanol desorption solution;

(2) concentrating a mixed solution of the effluent and the acid-water desorption solution obtained in the step (1) to produce a first concentrate; adding $\alpha$-1,4-glucosidase to the first concentrate, and heating to allow hydrolysis to produce a hydrolysis system; and adjusting a pH of the hydrolysis system to 3 for inactivating the $\alpha$-1,4-glucosidase to produce a hydrolysate;

(3) adding a steviol glycoside to the hydrolysate, and adjusting a pH of a resulting solution to 6.0; adding a glucosyltransferase, and heating to allow a reaction; after the reaction is completed, further heating for inactivating the glucosyltransferase to produce a reaction solution; concentrating the reaction solution until a solid content is 40 wt% to 50 wt% to produce a second concentrate; and spray-drying the second concentrate to produce high-purity GSG A; and

(4) concentrating the high-purity ethanol desorption solution until a solid content is 40 wt% to 50 wt% to produce a third concentrate, and spray-drying the third concentrate to produce high-purity GSG B.

As a preferred embodiment of the above technical solution, in the step (1), a concentration of the ethanol solution is 5 wt% to 15 wt%; and a concentration of GSGs in the GSG-containing ethanol solution is 30 g/L to 50 g/L.

As a preferred embodiment of the above technical solution, in the step (1), during the adsorption treatment, a flow rate of the GSG-containing ethanol solution is 0.25 BV/h to 1 BV/h.

As a preferred embodiment of the above technical solution, in the step (1), the acid aqueous solution is a hydrochloric acid solution with a concentration of 0.04 wt% to 0.06 wt%; and a concentration of the high-purity ethanol solution is 70 wt% to 80 wt%.

As a preferred embodiment of the above technical solution, in the step (1), during the desorption, a volume of the acid aqueous solution is 2 BV to 3 BV, a flow rate of the acid aqueous solution is 2 BV/h to 3 BV/h, a volume of the high-purity ethanol solution is 2 BV to 3 BV, and a flow rate of the high-purity ethanol solution is 2 BV/h to 3 BV/h.

As a preferred embodiment of the above technical solution, in the step (1), a volume of the effluent is 1 BV to 2 BV of a volume of the macroporous adsorption resin, and a volume of the acid-water desorption solution is 2 BV to 3 BV of the volume of the macroporous adsorption resin.

As a preferred embodiment of the above technical solution, in the step (2), a concentration factor for the concentrating is 3 to 5.

As a preferred embodiment of the above technical solution, in the step (2), 0.03 mL to 0.1 mL of the $\alpha$-1,4-glucosidase is added per 100 g of the crude GSG product.

As a preferred embodiment of the above technical solution, in the step (2), the hydrolysis is conducted at 40°C to 50°C for 1 h to 3 h.

As a preferred embodiment of the above technical solution, in the step (3), the steviol glycoside is STV; and 0.3 mL to 0.5 mL of the glucosyltransferase is added per 100 g of the crude GSG product.

As a preferred embodiment of the above technical solution, in the step (3), the reaction is conducted at 70°C to 80°C for 6 h to 10 h; and the glucosyltransferase is inactivated at 95°C.

Due to the above technical solutions, the present disclosure has the following beneficial effects:

In the present disclosure, a crude GSG product is first dissolved in an ethanol solution, then subjected to an adsorption treatment with a macroporous adsorption resin, and then subjected to a desorption treatment with an acid aqueous solution and a high-purity ethanol solution successively. The macroporous adsorption resin is an efficient adsorption material with a large specific surface area and a strong adsorption capacity, and can effectively adsorb a target substance with excellent selectivity for other impurities. The action of the macroporous adsorption resin can achieve the separation of GSGs from impurities, thereby increasing the purity. After the adsorption treatment of the macroporous adsorption resin, the acid aqueous solution can desorb dextrin adsorbed on the macroporous adsorption resin, and the high-purity ethanol solution can desorb GSGs from the macroporous adsorption resin, which increases the purity of GSGs and removes the residual impurities.

In the present disclosure, an effluent produced after the adsorption by the macroporous adsorption resin is mixed with an acid-water desorption solution, $\alpha$-1,4-glucosidase is added to allow hydrolysis to produce a hydrolysis system, and the hydrolysis system is mixed with a steviol glycoside to allow a reaction to produce high-purity GSGs. The dextrin recovered by the present disclosure is reused to prepare high-purity GSGs, which reduces the emission of waste liquids, lowers the energy consumption and cost of production of GSGs, and increases the yield of high-purity GSGs.

The method provided by the present disclosure involves relatively-simple operations, and can achieve the adsorptive separation of GSGs without complex equipment and conditions. The macroporous adsorption resin has a high adsorption capacity and regeneration capacity, and can be recycled, which can not only improve the efficiency of purification, but also reduce the cost.

To solve the above technical problems, the present disclosure adopts the following technical solutions:

A method for separating and purifying GSG and recovering glucose is provided, including the following steps:

(1) re-dissolving a crude GSG product with purified water to prepare a raw material solution;

(2) concentrating the raw material solution through nanofiltration, and collecting a retentate and a permeate;

(3) removing solvents in the retentate and the permeate separately to produce an intermediate A and an intermediate B, respectively;

(4) mixing the intermediate A with methanol, and allowing a settlement; conducting vacuum filtration to produce a first filter cake and a first filtrate; and drying the first filtrate to produce high-purity GSG;

(5) mixing the intermediate B with the first filter cake, adding purified water, and stirring until the intermediate B and the first filter cake are fully dissolved; adding $\alpha$-1,4-glucosidase, and allowing enzymatic hydrolysis; and after the enzymatic hydrolysis is completed, heating for inactivating the $\alpha$-1,4-glucosidase to produce a glucose-containing solution; and

(6) subjecting the glucose-containing solution to high-temperature concentration to produce a paste-like intermediate C; and slowly cooling the paste-like intermediate C for crystallization, and conducting vacuum filtration to produce a second filtrate and a second filter cake, where the second filtrate is recovered for subsequent production and the second filter cake is high-purity glucose.

As a preferred technical solution, in the step (1), a solid content of the raw material solution is 1 wt% to 5 wt%.

As a preferred technical solution, in the step (2), a nanofiltration membrane adopted for the nanofiltration has molecular weight cut-off of 500 Da to 800 Da, and an operating pressure for the nanofiltration is 3.5 bar to 5 bar.

As a preferred technical solution, in the step (3), the solvents in the retentate and the permeate are removed separately at a temperature of 80°C and a pressure of -0.1 MPa.

As a preferred technical solution, in the step (4), a concentration of the methanol is 95 wt% to 99 wt%, and a mass ratio of the intermediate A to the methanol is 1:(5-10).

As a preferred technical solution, in the step (4), the settlement lasts for 2 h to 8 h.

As a preferred technical solution, in the step (5), an amount of the $\alpha$-1,4-glucosidase added is 0.2% to 0.3% of a total weight of the intermediate B and the first filter cake.

As a preferred technical solution, in the step (5), the enzymatic hydrolysis is conducted for 5 h to 7 h at a temperature of 35°C to 45°C and a pH of 4.0 to 4.5; and the heating is conducted to 90°C for inactivating the $\alpha$-1,4-glucosidase.

As a preferred technical solution, in the step (6), the high-temperature concentration is conducted at 80°C to 90°C, and a solid content of the paste-like intermediate C is 70 wt% to 75 wt%.

As a preferred technical solution, in the step (6), the slow cooling is conducted at a rate of 10°C/h to 15°C/h to 20°C, and then the crystallization is conducted at 20°C for 5 h to 7 h.

Due to the above technical solutions, the present disclosure has the following beneficial effects:

In the present disclosure, a crude GSG product is re-dissolved in water, and then concentration is conducted with a nanofiltration membrane for preliminary purification. The unreacted RA and glucose and the GSG product in a system have significantly different relative molecular masses. Thus, GSGs can be separated with a 500 Da to 800 Da nanofiltration membrane to a large extent. Then a retentate is concentrated for solvent removal, and methanol is added for further purification to produce high-purity GSGs.

In the present disclosure, a permeate collected during the nanofiltration process is subjected to solvent removal and then mixed with a filter cake produced during the flocculent settling process. Then $\alpha$-1,4-glucosidase is added to allow enzymatic hydrolysis. Concentration and cooling crystallization are then conducted under appropriate conditions to produce high-purity glucose.

In summary, the method of the present disclosure involves a simple process and easy operations. The method achieves the purification of GSGs to produce a high-purity GSG product. In addition, with this method, the unreacted substrate can be largely recovered and reused, which reduces the production cost.

## DETAILED DESCRIPTION OF THE EMBODIMENTS

The present disclosure will be further described below with reference to embodiments. It should be understood that these embodiments are only intended to describe the present disclosure, rather than to limit the scope of the present disclosure.

The present disclosure provides a method for preparing GSG with MLS, including the following steps:

(1) The MLS, maltodextrin, and water are mixed and stirred until the MLS and the maltodextrin are fully dissolved. An enzyme is added, and enzymatic hydrolysis is allowed to produce an enzymatic hydrolysate.

(2) Activated carbon is added to the enzymatic hydrolysate obtained in the step (1) for impurity removal to produce an impurity-free solution. The impurity-free solution is subjected to enzyme inactivation and then filtration to produce a filtrate. The filtrate is subjected to an adsorption treatment with a macroporous adsorption resin.

(3) Desorption is conducted with an acid-alcohol solution that is produced from an ethanol solution and a hydrochloric acid solution and has a pH of 1 to 3 to produce a desorption solution.

(4) A pH of the desorption solution is adjusted to 5 to 6, and concentration is conducted with a nanofiltration membrane to produce a concentrate.

(5) A taste modifier is added to the concentrate, and spray-drying is conducted to produce the GSG.

The MLS in the present disclosure is a mother liquor produced after solution extraction of stevia leaves and crystallization. Generally, the MLS has a total glycoside content of 60% to 80%, where a content of RA is 20% to 40%, a content of STV is 10% to 25%, a content of RC is 10% to 20%, and a content of other glycosides is 5% to 20%.

In the present disclosure, the MLS, maltodextrin, and water are mixed for dissolution, an enzyme is added, and enzymatic hydrolysis is allowed to produce an enzymatic hydrolysate.

In the present disclosure, the MLS, the maltodextrin, and the water are in a mass ratio preferably of 1:(1-3):(5-10). The present disclosure has no special requirements for a source of the maltodextrin, and a commercially-available product can be adopted. A DE value of the maltodextrin in the present disclosure is preferably 5 to 10. The present disclosure has no special requirements for the water, and purified water is preferably adopted. During the mixing for dissolution, the present disclosure further includes stirring. The stirring is conducted at a rate preferably of 10 rpm and 100 rpm.

In the present disclosure, the enzymatic hydrolysis is started preferably after the MLS and the maltodextrin are completely dissolved. Further, the enzyme is preferably a mixture of a glucosyltransferase and an amylase, and the glucosyltransferase is preferably α-cyclodextrin glucosyltransferase. The lower the DE value of maltodextrin, the lower the degree of hydrolysis for dextrin. The more the substances such as dextrin and polysaccharides, the higher the viscosity of a product. The combined addition of a glucosyltransferase and an amylase in the present disclosure can accelerate the hydrolysis of dextrin and the transfer of glucosyl, and improve the conversion rate.

In the present disclosure, specifically, the glucosyltransferase is added preferably in a form of a glucosyltransferase solution. A concentration of the glucosyltransferase solution is preferably 0.8 wt% to 1.2 wt% and further preferably 1 wt%. An activity of the glucosyltransferase is preferably 100 U/g. The amylase is added preferably in a form of an amylase solid. A concentration of the amylase solid is preferably 0.4 wt% to 0.6 wt% and further preferably 0.5 wt%. An activity of the amylase is preferably 1,000 U/g to 3,000 U/g.

In the present disclosure, 0.5 mL to 2 mL of the glucosyltransferase solution is preferably added per 100 g of the MLS, and 0.1 g to 1 g of the amylase solid is preferably added per 100 g of the MLS.

Further, the enzymatic hydrolysis in the present disclosure is preferably conducted for 36 h to 48 h at a temperature of 60°C to 80°C and a pH of 5.5 to 6.0. In addition, the present disclosure has no special requirements for a reagent to adjust the pH, and a potassium hydroxide solution or a sodium hydroxide solution is preferably adopted. The present disclosure has no special restriction on a concentration of the potassium hydroxide solution or the sodium hydroxide solution in the present disclosure. For example, a concentration of the potassium hydroxide solution can be 1 v/v% to 10 v/v%, and a concentration of the sodium hydroxide solution can be 1 v/v% to 10 v/v%.

In the present disclosure, after the enzymatic hydrolysis is completed, activated carbon is added to an enzymatic hydrolysate for impurity removal. Based on the adsorption of the activated carbon, impurities such as flavones, saponins, and alkaloids in the enzymatic hydrolysate can be effectively adsorbed. The activated carbon also has a specific deodorizing effect, and can partially remove the bitterness of GSGs. Further, an amount of the activated carbon added is preferably 1% to 3% of a mass of the enzymatic hydrolysate, and the impurity removal is allowed preferably for 1 h to 6 h. The present disclosure has no special requirements for a pore size of the activated carbon. For example, the pore size of the activated carbon can be 2 nm to 50 nm. The present disclosure has no special requirements for a source of the activated carbon, and a commercially-available product may be adopted.

In the present disclosure, after the impurity removal is conducted with the activated carbon to produce an impurity-free solution, the impurity-free solution is subjected to enzyme inactivation and then filtration to produce a filtrate. Further, the enzyme inactivation is preferably high-temperature inactivation, and is conducted at a temperature preferably of 100°C to 120°C. The present disclosure has no special restriction on a time of the enzyme inactivation. Generally, when a temperature reaches 100°C to 120°C, the enzyme has been inactivated. Further, the filtration is conducted preferably with a plate and frame filter, where a pore size of a filter cloth is preferably 30 μm to 50 μm and an operating pressure is preferably 0.5 MPa to 2.5 MPa.

The enzyme inactivation for the impurity-free solution in the present disclosure is intended to terminate the enzymatic hydrolysis, thereby preventing the enzyme from affecting the final product and guaranteeing the quality of a product. The filtration with the plate and frame filter in the present disclosure is intended to filter both the activated carbon undergoing adsorption and the inactivated enzyme out.

In the present disclosure, the filtrate is subjected to adsorption with a macroporous adsorption resin. The present disclosure has no special restriction on a type of the macroporous adsorption resin, and the macroporous adsorption resin is preferably one selected from the group consisting of SD-9, AB-8, LX-T81, LX-T83, and LX-T28. The present disclosure has no special requirements for a source of the resin, and a commercially-available product can be adopted. The present disclosure has no special restriction on a manner for feeding the filtrate, and the feeding can be achieved by pumping, for example. The present disclosure has no special requirements for a support of the macroporous adsorption resin, and a resin column can be adopted, for example. Specifically, during the feeding, a feed amount of the filtrate is preferably 30% to 60% of a volume of a macroporous adsorption resin, and a feed rate of the filtrate is preferably 0.5 BV/h to 2 BV/h. In the present disclosure, an effluent produced after the adsorption of the macroporous adsorption resin includes specified amounts of dextrin and glucose, and can be further recycled to prepare other valuable by-products.

Further, after the filtrate is subjected to adsorption with the macroporous adsorption resin, a macroporous adsorption resin undergoing adsorption is washed with purified water, where preferably, an amount of the purified water is 1 BV to 3 BV of a volume of the macroporous adsorption resin undergoing adsorption, and a flow rate of the purified water is preferably 1 BV/h to 3 BV/h. The washing with the purified water in the present disclosure is intended to adsorb the residual filtrate in a resin column through the macroporous adsorption resin, thereby achieving the complete adsorption of the filtrate as much as possible and improving the adsorption rate.

In the present disclosure, after the adsorption with the macroporous adsorption resin, a macroporous adsorption resin undergoing adsorption is subjected to desorption, and a pH of an effluent is adjusted to 5 to 6 to produce a desorption solution. Specifically, a desorption agent adopted for the desorption is an acid-alcohol solution. A preparation method of the acid-alcohol solution includes: hydrochloric acid at a concentration of 0.3 wt% to 0.5 wt% is added to an ethanol solution with a concentration of 40 v/v% to 60 v/v%, and a pH is adjusted to 1 to 3 to produce the acid-alcohol solution. During the desorption, an amount of the acid-alcohol solution is preferably 2 BV to 4 BV of a volume of the macroporous adsorption resin undergoing adsorption, and a flow rate of the acid-alcohol solution is preferably 1 BV/h to 3 BV/h. The present disclosure has no special restriction on a pH-adjusting agent for adjusting the pH, and a pH-adjusting agent commonly used in the prior art can be adopted. In the present disclosure, the pH of the effluent produced after the desorption is adjusted to 5 to 6, such that glycosides can be prevented from being destructed due to the excessive acidity during the subsequent concentration process.

In the present disclosure, the desorption solution is concentrated through a nanofiltration membrane to produce a concentrate. Molecular weight cut-off of the nanofiltration membrane is preferably 400 Da to 500 Da. A solid content of the concentrate is 30 wt% to 50 wt%.

In the present disclosure, after the concentrate is obtained, a taste modifier is added to the concentrate, and spray-drying is conducted to produce the GSG. Specifically, the taste modifier is a composition of erythritol, sodium alginate, and valine in a mass ratio of preferably 2:(2-4):(5-8) and further preferably 2:3:5. An amount of the taste modifier added is preferably 0.1% to 0.5% of a mass of a product in the concentrate. The taste modifier adopted in the present disclosure is added preferably in a solid form. The addition of the taste modifier in the present disclosure is intended to adjust the taste of the product and reduce the bitterness of the product. In the present disclosure, the spray-drying is conducted with an inlet air temperature of 180°C to 200°C and an outlet air temperature of 80°C to 100°C.

In order to further illustrate the present disclosure, the method for preparing GSG with MLS provided by the present disclosure is described in detail below in connection with examples, but these examples should not be construed as limiting the claimed scope of the present disclosure. It should be noted that the raw materials adopted in the following examples and comparative examples all are commercially-available raw materials, unless otherwise specified. The main components of the MLS from the production of stevia glycosides in the following examples are shown in Table 1. These batches of MLS from the production of stevia glycosides all are provided by Zhucheng Haotian Pharm Co., Ltd.

Table 1 Steviol glycoside components of MLS in batches 1 to 4

| Batch | Moisture content/% | Reb A/% | STV/% | Reb C/% | Total glycoside content/% |
|---|---|---|---|---|---|
| 1 | 3.1 | 25 | 11 | 13 | 62 |
| 2 | 3.6 | 24 | 15 | 18 | 68 |
| 3 | 4.0 | 27 | 16 | 20 | 73 |
| 4 | 3.2 | 39 | 12 | 17 | 80 |

Example 1

S1: 100 g of MLS of batch 1 and 150 g of maltodextrin with a DE value of 8 were added to 700 mL of water, and stirring was conducted at a rotational speed of 50 rpm until the MLS and the maltodextrin were completely dissolved. 0.5 mL of $\alpha$-cyclodextrin glucosyltransferase (activity: 100 U/g) and 0.1 g of an amylase (activity: 1,000 U/g to 3,000 U/g) were added, and enzymatic hydrolysis was allowed for 42 h at a temperature of 70°C and a pH of 5.8 to produce 850 mL of an enzymatic hydrolysate.

S2: 10 g of activated carbon with a pore size of 30 nm was added to 850 mL of the enzymatic hydrolysate, and impurity removal was allowed for 3 h to produce 830 mL of an impurity-free solution.

S3: The impurity-free solution was heated to 110°C for enzyme inactivation, and then filtered by a plate and frame filter in which a filter cloth had a pore size of 40 $\mu$m under an operating pressure of 2.0 MPa to produce 815 mL of a filtrate.

S4: 815 mL of the filtrate was fed at a flow rate of 1 BV/h into a resin column packed with 2 L of a macroporous adsorption resin (SD-9) and subjected to an adsorption treatment.

S5: 2 L of a macroporous adsorption resin undergoing adsorption was subjected to desorption with an acid-alcohol solution. An amount of the acid-alcohol solution was 4 BV of a volume of the macroporous adsorption resin undergoing adsorption, and a flow rate of the acid-alcohol solution was 2 BV/h. Then, a pH of an effluent was adjusted to 5 to produce 7.8 L of a desorption solution. The acid-alcohol solution was a mixed solution of a 40 v/v% ethanol solution and a 0.5 wt% hydrochloric acid solution, and had a pH of 2.

S6: 7.8 L of the desorption solution was concentrated through a nanofiltration membrane with molecular weight cut-off of 400 Da, and a retentate was collected to obtain 350 mL of a concentrate with a solid content of 40 wt%.

S7: 0.14 g of a taste modifier (0.028 g of erythritol, 0.042 g of sodium alginate, and 0.07 g of valine) was added to 350 mL of the concentrate for modification. Spray-drying was conducted with an inlet air temperature of 190°C and an outlet air temperature of 90°C to produce 113 g of a GSG product.

Example 2

S1: 100 g of MLS of batch 2 and 100 g of maltodextrin with a DE value of 5 were added to 500 mL of water, and stirring was conducted at a rotational speed of 10 rpm until the MLS and the maltodextrin were completely dissolved. 1 mL of α-cyclodextrin glucosyltransferase (activity: 100 U/g) and 0.3 g of an amylase (activity: 1,000 U/g to 3,000 U/g) were added, and enzymatic hydrolysis was allowed for 48 h at a temperature of 80°C and a pH of 5.5 to produce 750 mL of an enzymatic hydrolysate.

S2: 15 g of activated carbon with a pore size of 2 nm was added to 750 mL of the enzymatic hydrolysate, and impurity removal was allowed for 6 h to produce 730 mL of an impurity-free solution.

S3: The impurity-free solution was heated to 120°C for enzyme inactivation, and then filtered by a plate and frame filter in which a filter cloth had a pore size of 50 μm under an operating pressure of 0.5 MPa to produce 720 mL of a filtrate.

S4: 720 mL of the filtrate was fed at a flow rate of 0.5 BV/h into a resin column packed with 2.4 L of a macroporous adsorption resin (AB-8) and subjected to an adsorption treatment.

S5: 2.4 L of a macroporous adsorption resin undergoing adsorption was subjected to desorption with an acid-alcohol solution. An amount of the acid-alcohol solution was 3 BV of a volume of the macroporous adsorption resin undergoing adsorption, and a flow rate of the acid-alcohol solution was 1 BV/h. Then, a pH of an effluent was adjusted to 5.5 to produce 7 L of a desorption solution. The acid-alcohol solution was a mixed solution of a 50 v/v% ethanol solution and a 0.5 wt% hydrochloric acid solution, and had a pH of 2.

S6: 7 L of the desorption solution was concentrated through a nanofiltration membrane with molecular weight cut-off of 450 Da, and a retentate was collected to obtain 430 mL of a concentrate with a solid content of 35 wt%.

S7: 0.45 g of a taste modifier (0.09 g of erythritol, 0.135 g of sodium alginate, and 0.225 g of valine) was added to 430 mL of the concentrate for modification. Spray-drying was conducted with an inlet air temperature of 200°C and an outlet air temperature of 80°C to produce 126 g of a GSG product.

Example 3

S1: 100 g of MLS of batch 3 and 200 g of maltodextrin with a DE value of 10 were added to 800 mL of water, and stirring was conducted at a rotational speed of 100 rpm until the MLS and the maltodextrin were completely dissolved. 1.5 mL of α-cyclodextrin glucosyltransferase (activity: 100 U/g) and 0.7 g of an amylase (activity: 1,000 U/g to 3,000 U/g) were added, and enzymatic hydrolysis was allowed for 36 h at a temperature of 60°C and a pH of 6.0 to produce 1,200 mL of an enzymatic hydrolysate.

S2: 20 g of activated carbon with a pore size of 50 nm was added to 1,200 mL of the enzymatic hydrolysate, and impurity removal was allowed for 1 h to produce 1,900 mL of an impurity-free solution.

S3: The impurity-free solution was heated to 100°C for enzyme inactivation, and then filtered by a plate and frame filter in which a filter cloth had a pore size of 30 μm under an operating pressure of 2.5 MPa to produce 1,800 mL of a filtrate.

S4: 1,800 mL of the filtrate was fed at a flow rate of 2 BV/h into a resin column packed with 3 L of a macroporous adsorption resin (T81) and subjected to an adsorption treatment.

S5: 3 L of a macroporous adsorption resin undergoing adsorption was subjected to desorption with an acid-alcohol solution. An amount of the acid-alcohol solution was 2 BV of a volume of the macroporous adsorption resin undergoing adsorption, and a flow rate of the acid-alcohol solution was 3 BV/h. Then, a pH of an effluent was adjusted to 6 to produce 6 L of a desorption solution. The acid-alcohol solution was a mixed solution of a 60 v/v% ethanol solution and a 0.5 wt% hydrochloric acid solution, and had a pH of 2.

S6: 6 L of the desorption solution was concentrated through a nanofiltration membrane with molecular weight cut-off of 500 Da, and a retentate was collected to obtain 300 mL of a concentrate with a solid content of 50 wt%.

S7: 0.75 g of a taste modifier (0.15 g of erythritol, 0.225 g of sodium alginate, and 0.375 g of valine) was added to 300 mL of the concentrate for modification. Spray-drying was conducted with an inlet air temperature of 180°C and an outlet air temperature of 100°C to produce 131 g of a GSG product.

Example 4

S1: 100 g of MLS of batch 4 and 250 g of maltodextrin with a DE value of 6 were added to 1,000 mL of water, and stirring was conducted at a rotational speed of 80 rpm until the MLS and the maltodextrin were completely dissolved. 2 mL of α-cyclodextrin glucosyltransferase (enzyme concentration: 0.15%, and activity: 100 U/g) and 1 g of an amylase (activity: 1,000 U/g to 3,000 U/g) were added, and enzymatic hydrolysis was allowed for 40 h at a temperature of 70°C and a pH of 5.6 to produce 850 mL of an enzymatic hydrolysate.

S2: 25 g of activated carbon with a pore size of 20 nm was added to 850 mL of the enzymatic hydrolysate, and impurity removal was allowed for 2 h to produce 830 mL of an impurity-free solution.

S3: The impurity-free solution was heated to 100°C for enzyme inactivation, and then filtered by a plate and frame filter in which a filter cloth had a pore size of 40 μm under an operating pressure of 1.0 MPa to produce 815 mL of a filtrate.

S4: 815 mL of the filtrate was fed at a flow rate of 1.5 BV/h into a resin column packed with 1.5 L of a macroporous adsorption resin (T83) and subjected to an adsorption treatment. Then a macroporous adsorption resin undergoing adsorption was washed with 2 BV of purified water at a flow rate of 2 BV/h.

S5: 1.5 L of a washed macroporous adsorption resin was subjected to desorption with an acid-alcohol solution. An amount of the acid-alcohol solution was 4 BV of a volume of the washed macroporous adsorption resin, and a flow rate of the acid-alcohol solution was 1.5 BV/h. Then, a pH of an effluent was adjusted to 5.5 to produce 6 L of a desorption solution. The acid-alcohol solution was a mixed solution of a 60 v/v% ethanol solution and a 0.5 wt% hydrochloric acid solution, and had a pH of 2.

S6: 6 L of the desorption solution was concentrated through a nanofiltration membrane with molecular weight cut-off of 500 Da, and a retentate was collected to obtain 400 mL of a concentrate with a solid content of 40 wt%.

S7: 0.8 g of a taste modifier (0.16 g of erythritol, 0.24 g of sodium alginate, and 0.4 g of valine) was added to 400 mL of the concentrate for modification. Spray-drying was conducted with an inlet air temperature of 180°C and an outlet air temperature of 90°C to produce 145 g of a GSG product.

Comparative Example 1

This comparative example was the same as Example 1, except that, in the S2 of this comparative example, 2 L of a macroporous adsorption resin SD-300 was used instead of the activated carbon.

Comparative Example 2

This comparative example was the same as Example 1, except that, in this comparative example, impurity removal was conducted with activated carbon first, and then enzymatic hydrolysis was carried out.

Comparative Example 3

This comparative example was the same as Example 1, except that, in the S5 of this comparative example, desorption was conducted first with a hydrochloric acid solution at a pH of 2 and then with a 40 v/v% ethanol solution, and resulting two effluents were combined.

Comparative Example 4

This comparative example was the same as Example 1, except that, in the S5 of this comparative example, desorption was conducted first with a 40 v/v% ethanol solution and then with a hydrochloric acid solution at a pH of 2, and resulting two effluents were combined.

Comparative Example 5

This comparative example was the same as Example 1, except that, in the S5 of this comparative example, a pH of an effluent was adjusted to 9 to produce a desorption solution.

Comparative Example 6

This comparative example was the same as Example 2, except that, in this comparative example, the enzyme inactivation in the S3 was omitted.

Comparative Example 7

This comparative example was the same as Example 3, except that, in this comparative example, the macroporous adsorption resin (T81) was replaced with an equal amount of a T-28 resin in the S4.

Comparative Example 8

This comparative example was the same as Example 4, except that, in this comparative example, the taste modifier was replaced with an equal amount of sodium citrate in the S7.

1. Testing of qualities, purities, and tastes of products

For Examples 1 to 4 and Comparative Examples 1 to 8 of the present disclosure, a GSG content was determined by a method according to the national standard GB 2760-2014.

During a taste test, a sample was diluted to 500 ppm, and tested at a temperature of 29°C and a humidity of 50 RH%. Specifically, according to the provisions in GB/T 16291.2-2010, 10 professional sensory evaluators were selected to constitute a sensory evaluation team. 1 h before an evaluation experiment, the sensory evaluators were restricted from food and drink, and especially were restricted from eating food severely affecting the taste sense. Samples were scored by the sensory evaluators for bitterness on a 10-point scale. An average was taken as a final score. An evaluation standard sample was a 5 wt% sucrose solution. Test results were shown in Table 2.

Table 2

|  | Product mass, g | Total glycoside content, % | Taste, bitterness |
|---|---|---|---|
| Example 1 | 113 | 90.4 | 1.8 |
| Example 2 | 126 | 91.5 | 1.7 |
| Example 3 | 131 | 93.2 | 1.4 |
| Example 4 | 145 | 97.5 | 1.6 |
| Comparative Example 1 | 122 | 86.7 | 4.2 |
| Comparative Example 2 | 119 | 88.4 | 2.4 |
| Comparative Example 3 | 101 | 89.1 | 2.6 |
| Comparative Example 4 | 104 | 91 | 3.2 |
| Comparative Example 5 | 93 | 82.6 | 3.1 |
| Comparative Example 6 | 128 | 90.8 | 3.7 |
| Comparative Example 7 | 141 | 88.7 | 3.2 |
| Comparative Example 8 | 145 | 91.8 | 2.6 |

It can be seen from the test results in Table 2 that, in contrast to the comparative examples, the GSG prepared with MLS as a raw material under the optimized conditions in the present disclosure has a high yield, a high total glycoside content, and a very prominent taste.

2. Sensory evaluation experiments for sweeteners

In the present disclosure, four comparative experiments were set to determine the taste difference between the GSG prepared by the present disclosure and the steviol glycoside in the original MLS. Each sample was diluted to 500 ppm. This is mainly because the steviol glycoside products had a high sweetness intensity, and needed to be diluted for distinguishing and evaluation. The four comparative experiments were specifically as follows:

experiment 1: 500 ppm of MLS (total glycoside content: 68%);
experiment 2: 500 ppm of the GSG prepared in Example 1;
experiment 3: 500 ppm of MLS (total glycoside content: 80%); and
experiment 4: 500 ppm of the GSG prepared in Example 4.

An evaluation standard sample was a 5 wt% sucrose solution.
Test conditions: a temperature: 29°C, and a humidity: 50 RH%.
Test date: May 16, 2023.
According to the provisions in GB/T 16291.2-2010, 10 professional sensory evaluators were selected to constitute a sensory evaluation team.
Specifically, 1 h before an evaluation experiment, the sensory evaluators were restricted from food and drink, and especially were restricted from eating food severely affecting the taste sense.
Each sweetener was comprehensively scored by the sensory evaluators for various indexes such as sweetness intensity, sweetness onset rate, lingering sweetness, bitterness, astringency, off-taste, and overall preference. The sweetness onset rate reflected how quickly the sweetness was perceived, and the lingering sweetness reflected the duration of sweetness. The overall preference was rated on a 100-point scale (with the 5 wt% sucrose solution as the full score of 100), but other indexes all were rated on a 10-point scale. An average was taken as a final score. Test results of the taste evaluation were shown in Table 3 below. The larger the sensory evaluation score, the more pronounced the taste characteristic.

Table 3

| No. | Sweetness intensity | Sweetness onset rate | Lingering sweetness | Bitterness | Astringency | Off-taste | Overall preference | Average ranking |
|---|---|---|---|---|---|---|---|---|
| Experiment 1 | 2.7 | 2.2 | 2.1 | 5.1 | 4.8 | 3.6 | 47 | 4 |
| Experiment 2 | 4.8 | 4.3 | 2.8 | 1.8 | 1.6 | 1.3 | 71 | 2 |
| Experiment 3 | 3.2 | 2.7 | 2.8 | 4.3 | 3.9 | 3.2 | 52 | 3 |
| Experiment 4 | 5.2 | 4.7 | 3.0 | 1.6 | 1.3 | 0.9 | 76 | 1 |

It can be seen from the test results in Table 3 that the GSG prepared in the present disclosure is improved in all aspects of taste compared with the original MLS. In particular, the sweetness intensity and sweetness onset rate are significantly improved, and the bitterness and astringency of the original MLS are also significantly reduced.

In the following examples, in 100 g of a crude GSG product, a total steviol glycoside (TSG) content was 71 wt%, a GSG content was 59 wt%, and a dextrin content was 28 wt%.

Qualitative analysis method for a product: A glycoside product was qualitatively determined by liquid chromatography quadrupole time-of-flight mass spectrometry. Detection conditions were as follows: ACQUITY UPLC BEH HILIC amino chromatographic column; column temperature: 30°C; gradient elution: acetonitrile : water = 80:20 (2 min) to 50:50 (30 min) (v/v); injection volume: 1 $\mu$L; injection concentration: 5 mg/mL; flow rate: 0.3 mL/min; and mass spectrometry conditions: collision voltage: 6 eV; ionization mode: electrospray ionization (ESI); negative ion detection mode; and molecular weight range: 200 to 2,000.

Quantitative analysis method for a product: The analytical testing method for GSG specified in Supplement No. 8 to GB 2760-2014 issued by the National Health and Family Planning Commission of the People's Republic of China was adopted.

Example 5

S1: 100 g of a crude GSG product was dissolved in 2 L of a 10 wt% ethanol solution, and then subjected to adsorption with a macroporous adsorption resin. During the adsorption, a flow rate of a solution was controlled at 0.5 BV/h, and an effluent was collected. Then desorption was conducted with a 0.05 wt% hydrochloric acid aqueous solution in a volume of 2 BV and at a flow rate of 2 BV/h, and an acid-water desorption solution was collected. Desorption was conducted with a 70 wt% ethanol solution in a volume of 2 BV and at a flow rate of 2 BV/h, and a high-purity ethanol desorption solution was collected.

S2: The effluent and the acid-water desorption solution were mixed and concentrated to produce 500 mL of a concentrate. 0.05 mL of $\alpha$-1,4-glucosidase was added to the concentrate, and heating was conducted to 45°C to allow hydrolysis for 2 h to produce a hydrolysis system. A pH of the hydrolysis system was adjusted to 3 for inactivating the $\alpha$-1,4-glucosidase to produce a hydrolysate.

S3: 30 g of STV was added to 500 mL of the hydrolysate, and a pH of a resulting solution was adjusted to 6.0. Heating was conducted to 75°C, 0.3 mL of a glucosyltransferase was added, and a reaction was allowed for 8 h. After the reaction was completed, heating was further conducted to 95°C for inactivating the glucosyltransferase to produce a reaction solution. The reaction solution was concentrated until a solid content was 50 wt%, and then spray-dried to produce 60 g of high-purity GSG A.

S4: The high-purity ethanol desorption solution was concentrated until a solid content was 50 wt%, and then spray-dried to produce 69 g of high-purity GSG B.

According to test results, in the high-purity GSG A, a TSG content was 93 wt%, a GSG content was 77 wt%, and a dextrin content was 5.8 wt%. In the high-purity GSG B, a TSG content was 99 wt%, a GSG content was 92 wt%, and a dextrin content was 0.2 wt%.

Example 6

S1: 100 g of a crude GSG product was dissolved in 3 L of a 10 wt% ethanol solution, and then subjected to adsorption with a macroporous adsorption resin. During the adsorption, a flow rate of a solution was controlled at 0.75 BV/h, and an effluent was collected. Then desorption was conducted with a 0.05 wt% hydrochloric acid aqueous solution in a volume of 2 BV and at a flow rate of 2 BV/h, and an acid-water desorption solution was collected. Desorption was conducted with a 80 wt% ethanol solution in a volume of 3 BV and at a flow rate of 3 BV/h, and a high-purity ethanol desorption solution was collected.

S2: The effluent and the acid-water desorption solution were mixed and concentrated to produce 500 mL of a concentrate. 0.05 mL of $\alpha$-1,4-glucosidase was added to the concentrate, and heating was conducted to 50°C to allow hydrolysis for 2 h to produce a hydrolysis system. A pH of the hydrolysis system was adjusted to 3 for inactivating the $\alpha$-1,4-glucosidase to produce a hydrolysate.

S3: 30 g of STV was added to 500 mL of the hydrolysate, and a pH of a resulting solution was adjusted to 6.0. Heating was conducted to 75°C, 0.4 mL of a glucosyltransferase was added, and a reaction was allowed for 7.5 h. After the reaction was completed, heating was further conducted to 95°C for inactivating the glucosyltransferase to produce a reaction solution. The reaction solution was concentrated until a solid content was 40 wt%, and then spray-dried to produce 61 g of high-purity GSG A.

S4: The high-purity ethanol desorption solution was concentrated until a solid content was 40 wt%, and then spray-dried to produce 68 g of high-purity GSG B.

According to test results, in the high-purity GSG A, a TSG content was 93.5 wt%, a GSG content was 79.5 wt%, and a dextrin content was 5.7 wt%. In the high-purity GSG B, a TSG content was 99 wt%, a GSG content was 93 wt%, and a dextrin content was 0.2 wt%.

Example 7

S1: 100 g of a crude GSG product was dissolved in 2.5 L of a 10 wt% ethanol solution, and then subjected to adsorption with a macroporous adsorption resin. During the adsorption, a flow rate of a solution was controlled at 0.8 BV/h, and an effluent was collected. Then desorption was conducted with a 0.05 wt% hydrochloric acid aqueous solution in a volume of 3 BV and at a flow rate of 3 BV/h, and an acid-water desorption solution was collected. Desorption was conducted with a 75 wt% ethanol solution in a volume of 2 BV and at a flow rate of 2 BV/h, and a high-purity ethanol desorption solution was collected.

S2: The effluent and the acid-water desorption solution were mixed and concentrated to produce 500 mL of a concentrate. 0.05 mL of $\alpha$-1,4-glucosidase was added to the concentrate, and heating was conducted to 45°C to allow hydrolysis for 2 h to produce a hydrolysis system. A pH of the hydrolysis system was adjusted to 3 for inactivating the $\alpha$-1,4-glucosidase to produce a hydrolysate.

S3: 30 g of STV was added to 500 mL of the hydrolysate, and a pH of a resulting solution was adjusted to 6.0. Heating was conducted to 75°C, 0.5 mL of a glucosyltransferase was added, and a reaction was allowed for 8 h. After the reaction was completed, heating was further conducted to 95°C for inactivating the glucosyltransferase to produce a reaction solution. The reaction solution was concentrated until a solid content was 45 wt%, and then spray-dried to produce 61 g of high-purity GSG A.

S4: The high-purity ethanol desorption solution was concentrated until a solid content was 45 wt%, and then spray-dried to produce 68.5 g of high-purity GSG B.

According to test results, in the high-purity GSG A, a TSG content was 93 wt%, a GSG content was 78 wt%, and a dextrin content was 5.6 wt%. In the high-purity GSG B, a TSG content was 99.5 wt%, a GSG content was 93 wt%, and a dextrin content was 0.15 wt%.

In the following examples, a method for calculating a yield of GSG was as follows:

$$\text{yield } (\%) = (\text{actual yield of a target product/theoretical yield of the target product}) \times 100\%.$$

In the following examples, GSG refers to glucosylated steviol glycoside and TSG refers to total steviol glycoside.

Example 8

S1: 100 g of a crude GSG product (TSG content: 85 wt%, GSG content: 79 wt%, and dextrin content: 14.5 wt%) resulting from a reaction was taken, 5 L of purified water was added, and stirring was conducted at room temperature and atmospheric pressure until the solid was dissolved to produce a raw material solution with a solid content of 2 wt%.

S2: The raw material solution was concentrated through a nanofiltration membrane with molecular weight cut-off of 800 Da under an operating pressure of 4 bar, and a retentate and a permeate were collected separately.

S3: Solvents in the retentate and the permeate obtained in the S2 were removed separately by a rotary evaporator at 80°C and -0.1 MPa to produce 85 g of an intermediate A and 15 g of an intermediate B, respectively.

S4: 85 g of the intermediate A was taken. 850 mL of methanol at a concentration of 99 wt% was added to a beaker, and the beaker was placed on a magnetic stirrer. The intermediate A was slowly added, and stirring was conducted continuously. After the intermediate A was completely dissolved, timing was started, and a settlement was allowed for 5 h. After the settlement was completed, vacuum filtration was conducted to produce a first filter cake and a first filtrate. The first filter cake was recovered for later use. A solvent in the first filtrate was removed to produce 83.3 g of a high-purity GSG product. In the high-purity GSG product, a GSG content was 92.3 wt% (namely, a purity of the product), a total glycoside content was 98 wt%, and a dextrin content was lower than 0.5 wt%. A GSG yield was 97.3%.

S5: The intermediate B was combined with the first filter cake obtained in the S4 to produce 16.7 g of a solid sample. The solid sample was mixed with purified water in a mass ratio of 1:10. $\alpha$-1,4-glucosidase was added at an amount 0.2% of a weight of the solid sample, and enzymatic hydrolysis was allowed for 6 h at a temperature of 40°C and a pH of 4.5. After the enzymatic hydrolysis was completed, a resulting enzymatic hydrolysis system was heated to 90°C for inactivating the $\alpha$-1,4-glucosidase to produce a glucose-containing solution.

S6: The glucose-containing solution was concentrated at 80°C until a solid content was 75 wt% to produce a paste-like intermediate C. The paste-like intermediate C was cooled to 20°C and kept at 20°C until a crystal was slowly precipitated, and allowed to stand for 6 h. Vacuum filtration was conducted to produce a second filter cake and a second filtrate. The second filtrate was recovered. The second filter cake was high-purity glucose with a purity of 80%.

Example 9

S1: 100 g of a crude GSG product (TSG content: 90 wt%, GSG content: 83.6 wt%, and dextrin content: 10 wt%) resulting from a reaction was taken, 10 L of purified water was added, and stirring was conducted at room temperature and atmospheric pressure until the solid was dissolved to produce a raw material solution with a solid content of 1 wt%.

S2: The raw material solution was concentrated through a nanofiltration membrane with molecular weight cut-off of 800 Da under an operating pressure of 4 bar, and a retentate and a permeate were collected separately.

S3: Solvents in the retentate and the permeate obtained in the S2 were removed separately by a rotary evaporator at 80°C and -0.1 MPa to produce 92 g of an intermediate A and 8 g of an intermediate B, respectively.

S4: 92 g of the intermediate A was taken. 850 mL of methanol at a concentration of 99 wt% was added to a beaker, and the beaker was placed on a magnetic stirrer. The intermediate A was slowly added, and stirring was conducted continuously. After the intermediate A was completely dissolved, timing was started, and a settlement was allowed for 8 h. After the settlement was completed, vacuum filtration was conducted to produce a first filter cake and a first filtrate. The first filter cake was recovered for later use. A solvent in the first filtrate was removed to produce 86 g of a high-purity GSG product. In the high-purity GSG product, a total glucoside content was 98.4 wt%, a GSG content was 92.9 wt%, and a dextrin content was lower than 0.6 wt%. A GSG yield was 95.6%.

S5: The intermediate B was combined with the first filter cake obtained in the S4 to produce 12.5 g of a solid sample. The solid sample was mixed with purified water in a mass ratio of 1:10. $\alpha$-1,4-glucosidase was added at an amount 0.3% of a weight of the solid sample, and enzymatic hydrolysis was allowed for 7 h at a temperature of 40°C and a pH of 4.5. After the enzymatic hydrolysis was completed, a resulting enzymatic hydrolysis system was heated to 90°C for inactivating the $\alpha$-1,4-glucosidase to produce a glucose-containing solution.

S6: The glucose-containing solution was concentrated at 90°C until a solid content was 70 wt% to produce a paste-like intermediate C. The paste-like intermediate C was cooled to 20°C and kept at 20°C until a crystal was slowly precipitated, and allowed to stand for 7 h. Vacuum filtration was conducted to produce a second filter cake and a second filtrate. The second filtrate was recovered. The second filter cake was high-purity glucose with a purity of 83%.

Example 10

S1: 100 g of a crude GSG product (TSG content: 85 wt%, GSG content: 79 wt%, and dextrin content: 14.5 wt%) resulting from a reaction was taken, 5 L of purified water was added, and stirring was conducted at room temperature and atmospheric pressure until the solid was dissolved to produce a raw material solution with a solid content of 2 wt%.

S2: The raw material solution was concentrated through a nanofiltration membrane with molecular weight cut-off of 700 Da under an operating pressure of 4 bar, and a retentate and a permeate were collected separately.

S3: Solvents in the retentate and the permeate obtained in the S2 were removed separately by a rotary evaporator at 80°C and -0.1 MPa to produce 83 g of an intermediate A and 16.3 g of an intermediate B, respectively.

S4: 83 g of the intermediate A was taken. 850 mL of methanol at a concentration of 99 wt% was added to a beaker, and the beaker was placed on a magnetic stirrer. The intermediate A was slowly added, and stirring was conducted continuously. After the intermediate A was completely dissolved, timing was started, and a settlement was allowed for 5 h.

After the settlement was completed, vacuum filtration was conducted to produce a first filter cake and a first filtrate. The first filter cake was recovered for later use. A solvent in the first filtrate was removed to produce 82.9 g of a high-purity GSG product. In the high-purity GSG product, a total glucoside content was 98 wt%, a GSG content was 93.1 wt%, and a dextrin content was lower than 0.5 wt%. A GSG yield was 97.7%.

S5: The intermediate B was combined with the first filter cake obtained in the S4 to produce 17 g of a solid sample. The solid sample was mixed with purified water in a mass ratio of 1:10. $\alpha$-1,4-glucosidase was added at an amount 0.2% of a weight of the solid sample, and enzymatic hydrolysis was allowed for 6 h at a temperature of 40°C and a pH of 4.5. After the enzymatic hydrolysis was completed, a resulting enzymatic hydrolysis system was heated to 90°C for inactivating the $\alpha$-1,4-glucosidase to produce a glucose-containing solution.

S6: The glucose-containing solution was concentrated at 80°C until a solid content was 75 wt% to produce a paste-like intermediate C. The paste-like intermediate C was cooled to 20°C and kept at 20°C until a crystal was slowly precipitated, and allowed to stand for 6 h. Vacuum filtration was conducted to produce a second filter cake and a second filtrate. The second filtrate was recovered. The second filter cake was high-purity glucose with a purity of 83%.

Example 11

S1: 100 g of a crude GSG product (TSG content: 85 wt%, GSG content: 79 wt%, and dextrin content: 14.5 wt%) resulting from a reaction was taken, 5 L of purified water was added, and stirring was conducted at room temperature and atmospheric pressure until the solid was dissolved to produce a raw material solution with a solid content of 2 wt%.

S2: The raw material solution was concentrated through a nanofiltration membrane with molecular weight cut-off of 600 Da under an operating pressure of 4 bar, and a retentate and a permeate were collected separately.

S3: Solvents in the retentate and the permeate obtained in the S2 were removed separately by a rotary evaporator at 80°C and -0.1 MPa to produce 83.9 g of an intermediate A and 15.5 g of an intermediate B, respectively.

S4: 83.9 g of the intermediate A was taken. 850 mL of methanol at a concentration of 99 wt% was added to a beaker, and the beaker was placed on a magnetic stirrer. The intermediate A was slowly added, and stirring was conducted continuously. After the intermediate A was completely dissolved, timing was started, and a settlement was allowed for 5 h. After the settlement was completed, vacuum filtration was conducted to produce a first filter cake and a first filtrate. The first filter cake was recovered for later use. A solvent in the first filtrate was removed to produce 80 g of a high-purity GSG product. In the high-purity GSG product, a total glucoside content was 99 wt%, a GSG content was 94.2 wt%, and a dextrin content was lower than 0.3 wt%. A GSG yield was 95.4%.

S5: The intermediate B was combined with the first filter cake obtained in the S4 to produce 16 g of a solid sample. The solid sample was mixed with purified water in a mass ratio of 1:10. $\alpha$-1,4-glucosidase was added at an amount 0.2% of a weight of the solid sample, and enzymatic hydrolysis was allowed for 6 h at a temperature of 40°C and a pH of 4.5. After the enzymatic hydrolysis was completed, a resulting enzymatic hydrolysis system was heated to 90°C for inactivating the $\alpha$-1,4-glucosidase to produce a glucose-containing solution.

S6: The glucose-containing solution was concentrated at 80°C until a solid content was 75 wt% to produce a paste-like intermediate C. The paste-like intermediate C was cooled to 20°C and kept at 20°C until a crystal was slowly precipitated, and allowed to stand for 6 h. Vacuum filtration was conducted to produce a second filter cake and a second filtrate. The second filtrate was recovered. The second filter cake was high-purity glucose with a purity of 82%.

Example 12

S1: 100 g of a crude GSG product (TSG content: 85 wt%, GSG content: 79 wt%, and dextrin content: 14.5 wt%) resulting from a reaction was taken, 5 L of purified water was added, and stirring was conducted at room temperature and atmospheric pressure until the solid was dissolved to produce a raw material solution with a solid content of 2 wt%.

S2: The raw material solution was concentrated through a nanofiltration membrane with molecular weight cut-off of 500 Da under an operating pressure of 4 bar, and a retentate and a permeate were collected separately.

S3: Solvents in the retentate and the permeate obtained in the S2 were removed separately by a rotary evaporator at 80°C and -0.1 MPa to produce 88.6 g of an intermediate A and 11 g of an intermediate B, respectively.

S4: 88 g of the intermediate A was taken. 850 mL of methanol at a concentration of 99 wt% was added to a beaker, and the beaker was placed on a magnetic stirrer. The intermediate A was slowly added, and stirring was conducted continuously. After the intermediate A was completely dissolved, timing was started, and a settlement was allowed for 5 h. After the settlement was completed, vacuum filtration was conducted to produce a first filter cake and a first filtrate. The first filter cake was recovered for later use. A solvent in the first filtrate was removed to produce 81.9 g of a high-purity GSG product. In the high-purity GSG product, a total glucoside content was 97.3 wt%, a GSG content was 91.2 wt%, and a dextrin content was lower than 1.0 wt%. A GSG yield was 94.5%.

S5: The intermediate B was combined with the first filter cake obtained in the S4 to produce 17 g of a solid sample. The solid sample was mixed with purified water in a mass ratio of 1:10. $\alpha$-1,4-glucosidase was added at an amount 0.2% of a weight of the solid sample, and enzymatic hydrolysis was allowed for 6 h at a temperature of 40°C and a pH of 4.5. After the enzymatic hydrolysis was completed, a resulting enzymatic hydrolysis system was heated to 90°C for inactivating the $\alpha$-1,4-glucosidase to produce a glucose-containing solution.

S6: The glucose-containing solution was concentrated at 80°C until a solid content was 75 wt% to produce a paste-like intermediate C. The paste-like intermediate C was cooled to 20°C and kept at 20°C until a crystal was slowly precipitated, and allowed to stand for 6 h. Vacuum filtration was conducted to produce a second filter cake and a second filtrate. The second filtrate was recovered. The second filter cake was high-purity glucose with a purity of 85%.

Example 13

S1: 100 g of a crude GSG product (TSG content: 85 wt%, GSG content: 79 wt%, and dextrin content: 14.5 wt%) resulting from a reaction was taken, 5 L of purified water was added, and stirring was conducted at room temperature and atmospheric pressure until the solid was dissolved to produce a raw material solution with a solid content of 2 wt%.

S2: The raw material solution was concentrated through a nanofiltration membrane with molecular weight cut-off of 800 Da under an operating pressure of 4 bar, and a retentate and a permeate were collected separately.

S3: Solvents in the retentate and the permeate obtained in the S2 were removed separately by a rotary evaporator at 80°C and -0.1 MPa to produce 84.6 g of an intermediate A and 15 g of an intermediate B, respectively.

S4: 84.6 g of the intermediate A was taken. 850 mL of methanol at a concentration of 99 wt% was added to a beaker, and the beaker was placed on a magnetic stirrer. The intermediate A was slowly added, and stirring was conducted continuously. After the intermediate A was completely dissolved, timing was started, and a settlement was allowed for 5 h. After the settlement was completed, vacuum filtration was conducted to produce a first filter cake and a first filtrate. The first filter cake was recovered for later use. A solvent in the first filtrate was removed to produce 83.5 g of a high-purity GSG product. In the high-purity GSG product, a total glucoside content was 97.8 wt%, a GSG content was 92.9 wt%, and a dextrin content was lower than 0.5 wt%. A GSG yield was 98.2%.

S5: The intermediate B was combined with the first filter cake obtained in the S4 to produce 16.5 g of a solid sample. The solid sample was mixed with purified water in a mass ratio of 1:10. $\alpha$-1,4-glucosidase was added at an amount 0.2% of a weight of the solid sample, and enzymatic hydrolysis was allowed for 8 h at a temperature of 45°C and a pH of 4.5. After the enzymatic hydrolysis was completed, a resulting enzymatic hydrolysis system was heated to 90°C for inactivating the $\alpha$-1,4-glucosidase to produce a glucose-containing solution.

S6: The glucose-containing solution was concentrated at 80°C until a solid content was 75 wt% to produce a paste-like intermediate C. The paste-like intermediate C was cooled to 20°C and kept at 20°C until a crystal was slowly precipitated, and allowed to stand for 6 h. Vacuum filtration was conducted to produce a second filter cake and a second filtrate. The second filtrate was recovered. The second filter cake was high-purity glucose with a purity of 84.3%.

Example 14

S1: 100 g of a crude GSG product (TSG content: 85 wt%, GSG content: 79 wt%, and dextrin content: 14.5 wt%) resulting from a reaction was taken, 5 L of purified water was added, and stirring was conducted at room temperature and atmospheric pressure until the solid was dissolved to produce a raw material solution with a solid content of 2 wt%.

S2: The raw material solution was concentrated through a nanofiltration membrane with molecular weight cut-off of 800 Da under an operating pressure of 4 bar, and a retentate and a permeate were collected separately.

S3: Solvents in the retentate and the permeate obtained in the S2 were removed separately by a rotary evaporator at 80°C and -0.1 MPa to produce 84.8 g of an intermediate A and 14.9 g of an intermediate B, respectively.

S4: 84 g of the intermediate A was taken. 850 mL of methanol at a concentration of 99 wt% was added to a beaker, and the beaker was placed on a magnetic stirrer. The intermediate A was slowly added, and stirring was conducted continuously. After the intermediate A was completely dissolved, timing was started, and a settlement was allowed for 5 h. After the settlement was completed, vacuum filtration was conducted to produce a first filter cake and a first filtrate. The first filter cake was recovered for later use. A solvent in the first filtrate was removed to produce 82.5 g of a high-purity GSG product. In the high-purity GSG product, a total glucoside content was 98.1 wt%, a GSG content was 92.9 wt%, and a dextrin content was lower than 0.7 wt%. A GSG yield was 97%.

S5: The intermediate B was combined with the first filter cake obtained in the S4 to produce 17 g of a solid sample. The solid sample was mixed with purified water in a mass ratio of 1:10. $\alpha$-1,4-glucosidase was added at an amount 0.2% of a weight of the solid sample, and enzymatic hydrolysis was allowed for 12 h at a temperature of 35°C and a pH of 4.5. After the enzymatic hydrolysis was completed, a resulting enzymatic hydrolysis system was heated to 90°C for inactivating the $\alpha$-1,4-glucosidase to produce a glucose-containing solution.

S6: The glucose-containing solution was concentrated at 80°C until a solid content was 75 wt% to produce a paste-like intermediate C. The paste-like intermediate C was cooled to 20°C and kept at 20°C until a crystal was slowly precipitated, and allowed to stand for 8 h. Vacuum filtration was conducted to produce a second filter cake and a second filtrate. The second filtrate was recovered. The second filter cake was high-purity glucose with a purity of 83.6%.

Example 15

S1: 100 g of a crude GSG product (TSG content: 85 wt%, GSG content: 79 wt%, and dextrin content: 14.5 wt%) resulting from a reaction was taken, 5 L of purified water was added, and stirring was conducted at room temperature and atmospheric pressure until the solid was dissolved to produce a raw material solution with a solid content of 2 wt%.

S2: The raw material solution was concentrated through a nanofiltration membrane with molecular weight cut-off of 800 Da under an operating pressure of 4 bar, and a retentate and a permeate were collected separately.

S3: Solvents in the retentate and the permeate obtained in the S2 were removed separately by a rotary evaporator at 80°C and -0.1 MPa to produce 86.1 g of an intermediate A and 13.8 g of an intermediate B, respectively.

S4: 86 g of the intermediate A was taken. 850 mL of methanol at a concentration of 99 wt% was added to a beaker, and the beaker was placed on a magnetic stirrer. The intermediate A was slowly added, and stirring was conducted continuously. After the intermediate A was completely dissolved, timing was started, and a settlement was allowed for 5 h. After the settlement was completed, vacuum filtration was conducted to produce a first filter cake and a first filtrate. The first filter cake was recovered for later use. A solvent in the first filtrate was removed to produce 84 g of a high-purity GSG product. In the high-purity GSG product, a total glucoside content was 98.5 wt%, a GSG content was 93.1 wt%, and a dextrin content was lower than 0.6 wt%. A GSG yield was 99%.

S5: The intermediate B was combined with the first filter cake obtained in the S4 to produce 15 g of a solid sample. The solid sample was mixed with purified water in a mass ratio of 1:10. $\alpha$-1,4-glucosidase was added at an amount 0.2% of a weight of the solid sample, and enzymatic hydrolysis was allowed for 6 h at a temperature of 30°C and a pH of 4. After the enzymatic hydrolysis was completed, a resulting enzymatic hydrolysis system was heated to 90°C for inactivating the $\alpha$-1,4-glucosidase to produce a glucose-containing solution.

S6: The glucose-containing solution was concentrated at 80°C until a solid content was 75 wt% to produce a paste-like intermediate C. The paste-like intermediate C was cooled to 20°C and kept at 20°C until a crystal was slowly precipitated, and allowed to stand for 6 h. Vacuum filtration was conducted to produce a second filter cake and a second filtrate. The second filtrate was recovered. The second filter cake was high-purity glucose with a purity of 80%.

In summary, in the examples of the present application, the glucose prepared has a purity as high as 85%, the GSG product prepared has a total glycoside content of 99 wt% and a GSG content of 94.2 wt%, and a GSG yield is as high as 99%.

In addition, it should be understood that various changes or modifications may be made to the present disclosure by those skilled in the art after reading the content of the present disclosure, and these equivalent forms also fall within the scope defined by the appended claims of the present application.

**Claims**

1. A method for preparing a glucosylated steviol glycoside (GSG) with mother liquor sugar (MLS), comprising the following steps:

   (1) mixing the MLS, maltodextrin, and water, and stirring until the MLS and the maltodextrin are fully dissolved; and adding an enzyme, and allowing enzymatic hydrolysis to produce an enzymatic hydrolysate;
   (2) adding activated carbon to the enzymatic hydrolysate obtained in the step (1) for impurity removal to produce an impurity-free solution; subjecting the impurity-free solution to enzyme inactivation and then filtration to produce a filtrate; and subjecting the filtrate to an adsorption treatment with a macroporous adsorption resin;
   (3) conducting desorption with an acid-alcohol solution that is produced from an ethanol solution and a hydrochloric acid solution and has a pH of 1 to 3 to produce a desorption solution;
   (4) adjusting a pH of the desorption solution to 5 to 6, and concentrating with a nanofiltration membrane to produce a concentrate; and
   (5) adding a taste modifier to the concentrate, and spray-drying to produce the GSG.

2. The method for preparing GSG with MLS according to claim 1, wherein in the step (1), the MLS, the maltodextrin, and the water are in a mass ratio of 1:(1-3):(5-10).

3. The method for preparing GSG with MLS according to claim 1, wherein in the step (1), the enzyme is a mixture of a glucosyltransferase and an amylase; the glucosyltransferase is $\alpha$-cyclodextrin glucosyltransferase; the glucosyl-transferase is added in a form of a glucosyltransferase solution with a concentration of 0.8 wt% to 1.2 wt%; the amylase is added in a form of an amylase solid with a concentration of 0.4 wt% to 0.6 wt%; and/or

0.5 mL to 2 mL of the glucosyltransferase solution is added per 100 g of the MLS, and 0.1 g to 1 g of the amylase solid is added per 100 g of the MLS.

4. The method for preparing GSG with MLS according to claim 1, wherein in the step (1), the enzymatic hydrolysis is conducted for 36 h to 48 h at a temperature of 60°C to 80°C and a pH of 5.5 to 6.0.

5. The method for preparing GSG with MLS according to claim 1, wherein in the step (2), an amount of the activated carbon added is 1% to 3% of a mass of the enzymatic hydrolysate, and the impurity removal lasts for 1 h to 6 h.

6. The method for preparing GSG with MLS according to claim 1, wherein in the step (2), when the adsorption treatment is conducted with the macroporous adsorption resin, a feed volume of the filtrate is 30% to 60% of a volume of the macroporous adsorption resin, and a feed rate of the filtrate is 0.5 BV/h to 2 BV/h.

7. The method for preparing GSG with MLS according to claim 6, wherein before the desorption for a macroporous adsorption resin undergoing adsorption, the method further comprises: washing the macroporous adsorption resin undergoing adsorption with purified water, wherein during the washing, an amount of the purified water is 1 BV to 3 BV of a volume of the macroporous adsorption resin undergoing adsorption, and a flow rate of the purified water is 1 BV/h to 3 BV/h.

8. The method for preparing GSG with MLS according to claim 1, wherein in the step (3), a concentration of the ethanol solution is 40 v/v% to 60 v/v% and a concentration of the hydrochloric acid solution is 0.3 wt% to 0.5 wt%; and during the desorption, a volume of the acid-alcohol solution is 2 BV to 4 BV of a volume of a macroporous adsorption resin undergoing adsorption, and a flow rate of the acid-alcohol solution is 1 BV/h to 3 BV/h.

9. The method for preparing GSG with MLS according to claim 1, wherein in the step (4), molecular weight cut-off of the nanofiltration membrane is 400 Da to 500 Da.

10. The method for preparing GSG with MLS according to claim 1, wherein in the step (5), the taste modifier is a composition of erythritol, sodium alginate, and valine in a mass ratio of 2:(2-4):(5-8); and an amount of the taste modifier added is 0.1% to 0.5% of a mass of a product in the concentrate.

11. A method for purifying GSG, comprising the following steps:

(1) dissolving a crude GSG product in an ethanol solution to produce a GSG-containing ethanol solution; subjecting the GSG-containing ethanol solution to an adsorption treatment with a macroporous adsorption resin, and collecting an effluent; and conducting desorption with an acid aqueous solution and a high-purity ethanol solution successively, and collecting an acid-water desorption solution and a high-purity ethanol desorption solution;
(2) concentrating a mixed solution of the effluent and the acid-water desorption solution obtained in the step (1) to produce a first concentrate; adding $\alpha$-1,4-glucosidase to the first concentrate, and heating to allow hydrolysis to produce a hydrolysis system; and adjusting a pH of the hydrolysis system to 3 for inactivating the $\alpha$-1,4-glucosidase to produce a hydrolysate;
(3) adding a steviol glycoside to the hydrolysate, and adjusting a pH of a resulting solution to 6.0; adding a glucosyltransferase, and heating to allow a reaction; after the reaction is completed, further heating for inactivating the glucosyltransferase to produce a reaction solution; concentrating the reaction solution until a solid content is 40 wt% to 50 wt% to produce a second concentrate; and spray-drying the second concentrate to produce high-purity GSG A; and
(4) concentrating the high-purity ethanol desorption solution until a solid content is 40 wt% to 50 wt% to produce a third concentrate, and spray-drying the third concentrate to produce high-purity GSG B.

12. The method for purifying GSG according to claim 11, wherein in the step (1), a concentration of the ethanol solution is 5 wt% to 15 wt%; and a concentration of GSGs in the GSG-containing ethanol solution is 30 g/L to 50 g/L.

13. The method for purifying GSG according to claim 11, wherein in the step (1), during the adsorption treatment, a flow rate of the GSG-containing ethanol solution is 0.25 BV/h to 1 BV/h.

14. The method for purifying GSG according to claim 11, wherein in the step (1), the acid aqueous solution is a hydrochloric acid solution with a concentration of 0.04 wt% to 0.06 wt%; a concentration of the high-purity ethanol solution is 70 wt% to 80 wt%; and during the desorption, a volume of the acid aqueous solution is 2 BV to 3 BV, a flow rate of the acid aqueous solution is 2 BV/h to 3 BV/h, a volume of the high-purity ethanol solution is 2 BV to 3 BV, and a flow rate of the high-purity ethanol solution is 2 BV/h to 3 BV/h.

15. The method for purifying GSG according to claim 11, wherein in the step (1), a volume of the effluent is 1 BV to 2 BV of a volume of the macroporous adsorption resin, and a volume of the acid-water desorption solution is 2 BV to 3 BV of the volume of the macroporous adsorption resin.

16. The method for purifying GSG according to claim 11, wherein in the step (2), a concentration factor for the concentrating is 3 to 5.

17. The method for purifying GSG according to claim 11, wherein in the step (2), 0.03 mL to 0.1 mL of the $\alpha$-1,4-glucosidase is added per 100 g of the crude GSG product.

18. The method for purifying GSG according to claim 11, wherein in the step (2), the hydrolysis is conducted at 40°C to 50°C for 1 h to 3 h.

19. The method for purifying GSG according to claim 11, wherein in the step (3), the steviol glycoside is stevioside (STV); and 0.3 mL to 0.5 mL of the glucosyltransferase is added per 100 g of the crude GSG product.

20. The method for purifying GSG according to claim 11, wherein in the step (3), the reaction is conducted at 70°C to 80°C for 6 h to 10 h; and the glucosyltransferase is inactivated at 95°C.

21. A method for separating and purifying GSG and recovering glucose, comprising the following steps:

(1) re-dissolving a crude GSG product with purified water to prepare a raw material solution;
(2) concentrating the raw material solution through nanofiltration, and collecting a retentate and a permeate;
(3) removing solvents in the retentate and the permeate separately to produce an intermediate A and an intermediate B, respectively;
(4) mixing the intermediate A with methanol, and allowing a settlement; conducting vacuum filtration to produce a first filter cake and a first filtrate; and drying the first filtrate to produce high-purity GSG;
(5) mixing the intermediate B with the first filter cake, adding purified water, and stirring until the intermediate B and the first filter cake are fully dissolved; adding $\alpha$-1,4-glucosidase, and allowing enzymatic hydrolysis; and after the enzymatic hydrolysis is completed, heating for inactivating the $\alpha$-1,4-glucosidase to produce a glucose-containing solution; and
(6) subjecting the glucose-containing solution to high-temperature concentration to produce a paste-like intermediate C; and slowly cooling the paste-like intermediate C for crystallization, and conducting vacuum filtration to produce a second filtrate and a second filter cake, wherein the second filtrate is recovered for subsequent production and the second filter cake is high-purity glucose.

22. The method for separating and purifying GSG and recovering glucose according to claim 21, wherein in the step (1), a solid content of the raw material solution is 1 wt% to 5 wt%.

23. The method for separating and purifying GSG and recovering glucose according to claim 21, wherein in the step (2), a nanofiltration membrane adopted for the nanofiltration has molecular weight cut-off of 500 Da to 800 Da, and an operating pressure for the nanofiltration is 3.5 bar to 5 bar.

24. The method for separating and purifying GSG and recovering glucose according to claim 21, wherein in the step (3), the solvents in the retentate and the permeate are removed separately at a temperature of 80°C and a pressure of -0.1 MPa.

25. The method for separating and purifying GSG and recovering glucose according to claim 21, wherein in the step (4), a concentration of the methanol is 95 wt% to 99 wt%, and a mass ratio of the intermediate A to the methanol is 1:(5-10).

26. The method for separating and purifying GSG and recovering glucose according to claim 21, wherein in the step (4), the settlement lasts for 2 h to 8 h.

27. The method for separating and purifying GSG and recovering glucose according to claim 21, wherein in the step (5), an

amount of the $\alpha$-1,4-glucosidase added is 0.2% to 0.3% of a total weight of the intermediate B and the first filter cake.

28. The method for separating and purifying GSG and recovering glucose according to claim 21, wherein in the step (5), the enzymatic hydrolysis is conducted for 5 h to 7 h at a temperature of 35°C to 45°C and a pH of 4.0 to 4.5; and the heating is conducted to 90°C for inactivating the $\alpha$-1,4-glucosidase.

29. The method for separating and purifying GSG and recovering glucose according to claim 21, wherein in the step (6), the high-temperature concentration is conducted at 80°C to 90°C, and a solid content of the paste-like intermediate C is 70 wt% to 75 wt%.

30. The method for separating and purifying GSG and recovering glucose according to claim 21, wherein in the step (6), the slow cooling is conducted at a rate of 10°C/h to 15°C/h to 20°C, and then the crystallization is conducted at 20°C for 5 h to 7 h.

## INTERNATIONAL SEARCH REPORT

| International application No. |
|---|
| **PCT/CN2024/106037** |

| A. | CLASSIFICATION OF SUBJECT MATTER |
|---|---|

C12P19/56(2006.01)i; C12P19/18(2006.01)i; C12P19/14(2006.01)i; C12P19/22(2006.01)i; C07H1/06(2006.01)i; C07H3/02(2006.01)i; C07H15/256(2006.01)i

According to International Patent Classification (IPC) or to both national classification and IPC

| B. | FIELDS SEARCHED |
|---|---|

Minimum documentation searched (classification system followed by classification symbols)

IPC:C12P,C07H

Documentation searched other than minimum documentation to the extent that such documents are included in the fields searched

Electronic data base consulted during the international search (name of data base and, where practicable, search terms used)

CNTXT, ENTXTC, DWPI, CNKI, ISI WEB OF SCIENCE: 葡萄糖基 s 甜菊糖苷, 麦芽糊精, 葡萄糖基转移酶, 淀粉酶, 葡萄糖水解酶, 解析, 回收, Glucosyl s steviol glycoside, maltodextrin, glucosyltransferase, amylase, glucohydrolase, resolving, recovering

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 117844886 A (DONGTAI HAORUI BIOTECHNOLOGY CO., LTD.) 09 April 2024 (2024-04-09)<br>claims 1-10 | 1-10 |
| PX | CN 116987132 A (DONGTAI HAORUI BIOTECHNOLOGY CO., LTD.) 03 November 2023 (2023-11-03)<br>claims 1-10 | 11-20 |
| PX | CN 117327131 A (DONGTAI HAORUI BIOTECHNOLOGY CO., LTD.) 02 January 2024 (2024-01-02)<br>claims 1-10 | 21-30 |
| X | CN 108727443 A (DONGTAI HAORUI BIOTECHNOLOGY CO., LTD.) 02 November 2018 (2018-11-02)<br>claims 1-3 and 12 | 1-10 |
| X | CN 108753871 A (DONGTAI HAORUI BIOTECHNOLOGY CO., LTD.) 06 November 2018 (2018-11-06)<br>claims 1-3 and 9 | 1-10 |

| ✓ Further documents are listed in the continuation of Box C. | ✓ See patent family annex. |
|---|---|

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **17 October 2024** | **22 October 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

International application No.

**PCT/CN2024/106037**

### C.    DOCUMENTS CONSIDERED TO BE RELEVANT

| Category* | Citation of document, with indication, where appropriate, of the relevant passages | Relevant to claim No. |
|---|---|---|
| A | KR 100888694 B1 (KIM KYUNG JAE et al.) 16 March 2009 (2009-03-16) abstract, and claims 1-14 | 1-30 |
| A | CN 111187315 A (SHANGHAI URANA BIOLOGICAL TECHNOLOGY CO., LTD.) 22 May 2020 (2020-05-22) entire document | 1-30 |
| A | CN 109770323 A (JINHE YIKANG (BEIJING) BIOTECHNOLOGY CO., LTD.) 21 May 2019 (2019-05-21) entire document | 1-30 |
| A | CN 105175462 A (HUNAN VIGOR BIO-TECH CO., LTD.) 23 December 2015 (2015-12-23) entire document | 1-30 |

Form PCT/ISA/210 (second sheet) (July 2022)

## INTERNATIONAL SEARCH REPORT
### Information on patent family members

International application No.

**PCT/CN2024/106037**

| Patent document cited in search report | | | Publication date (day/month/year) | Patent family member(s) | Publication date (day/month/year) |
|---|---|---|---|---|---|
| CN | 117844886 | A | 09 April 2024 | None | |
| CN | 116987132 | A | 03 November 2023 | None | |
| CN | 117327131 | A | 02 January 2024 | None | |
| CN | 108727443 | A | 02 November 2018 | None | |
| CN | 108753871 | A | 06 November 2018 | None | |
| KR | 100888694 | B1 | 16 March 2009 | None | |
| CN | 111187315 | A | 22 May 2020 | None | |
| CN | 109770323 | A | 21 May 2019 | None | |
| CN | 105175462 | A | 23 December 2015 | None | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202311848635
- CN 202310879842
- CN 202311239776
- WO 202010447914 A
- GB 27602014 A
- GB 1629122010 T